(19) Europäisches Patentamt / European Patent Office / Office européen des brevets

(11) **EP 2 623 096 B1**

(12) **FASCICULE DE BREVET EUROPEEN**

(45) Date de publication et mention de la délivrance du brevet:
**03.04.2019 Bulletin 2019/14**

(51) Int Cl.:
*A61K 9/50* $^{(2006.01)}$     *A61K 31/495* $^{(2006.01)}$

(21) Numéro de dépôt: **13153638.5**

(22) Date de dépôt: **01.02.2013**

(54) **Composition pharmaceutique a liberation prolongée de trimetazidine**

Pharmazeutische Trimetazidin-Retard-Zusammensetzung

Pharmaceutical composition with slow release of trimetazidine

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **03.02.2012 FR 1200322**

(43) Date de publication de la demande:
**07.08.2013 Bulletin 2013/32**

(73) Titulaire: **Les Laboratoires Servier**
**92284 Suresnes Cedex (FR)**

(72) Inventeurs:
- **Genty, Patrick**
  **45100 ORLEANS (FR)**
- **Hermelin, Christophe**
  **45380 CHAINGY (FR)**
- **Pean, Jean-Manuel**
  **45000 ORLEANS (FR)**

(56) Documents cités:
**EP-A1- 0 673 649**     **EP-A1- 1 108 424**
**EP-A1- 1 195 160**     **WO-A1-2010/086868**
**US-A1- 2011 274 751**

**EP 2 623 096 B1**

**Description**

L'invention a pour objet une forme pharmaceutique à libération prolongée de trimétazidine ainsi que son utilisation dans le traitement de l'angor.

La trimétazidine ou 1-(2,3,4-triméthoxybenzyl) pipérazine est une molécule qui, en préservant le métabolisme énergétique de la cellule exposée à l'hypoxie ou à l'ischémie, évite l'effondrement du taux intracellulaire de l'adénosinetriphosphate (ATP). Elle assure ainsi le fonctionnement des pompes ioniques et des flux transmembranaires sodium-potassium et maintient l'homéostasie cellulaire.

Le dichlorhydrate de trimétazidine est utilisé en thérapeutique pour le traitement prophylactique de la crise d'angine de poitrine, lors des atteintes choriorétiniennnes ainsi que pour le traitement des vertiges d'origine vasculaire (vertiges de Ménière, acouphènes).

L'utilisation de la trimétazidine en thérapeutique a été décrite sous forme de composition pharmaceutique à libération immédiate administrée trois fois par jour notamment dans le brevet FR 2 490 963.

Le brevet EP 1 108 424 décrit une forme à libération prolongée permettant de couvrir la totalité du nychthémère sur la base d'une administration deux fois par jour. Cette forme à libération prolongée permet d'obtenir après chaque prise des taux plasmatiques chez l'homme, supérieurs à 70 µg/l et de maintenir avant la prise suivante un taux plasmatique supérieur ou égal à 40 µg/l.

Des compositions pharmaceutiques à libération prolongée de trimétazidine de type réservoir assurant la libération du principe actif sur une période de 16 heures ont été décrites dans le brevet EP 0 673 649. Ces formes de type réservoir à administration journalière unique ont l'avantage de diminuer les pics sanguins tout en assurant des taux plasmatiques réguliers et constants de trimétazidine.

Dans l'arsenal thérapeutique mis à la disposition des patients, les formes à libération prolongée de trimétazidine s'avèrent nécessaire pour assurer une observance et une protection thérapeutique optimale du patient. Les formes à libération prolongée selon la présente invention permettent d'une part une libération progressive et soutenue sur 24 heures à une concentration plasmatique thérapeutiquement efficace de la trimétazidine et d'autre part une disponibilité de la trimétazidine à concentration plasmatique thérapeutiquement efficace à bref délai après l'administration. Par concentration plasmatique thérapeutiquement efficace de trimétazidine on entend un taux plasmatique supérieur ou égal à 40µg/l permettant une protection myocardique efficace. Par ailleurs, par bref délai on entend un délai inférieur à 4 heures, de préférence un délai inférieur à 3 heures.

La composition pharmaceutique selon l'invention s'applique à une administration orale une fois par jour et libère la trimétazidine tout au long du nycthémère tout en apportant une grande sécurité vis à vis de toute libération de type « burst » ou discontinue.

La composition pharmaceutique selon la présente invention est une composition à libération prolongée de trimétazidine dans laquelle la phase interne comprend un noyau neutre enrobé de trimétazidine et la couche externe comprend un retardant et un anti-agglomérant dans laquelle le pourcentage de retardant est compris entre 5.5 et 8% de la masse totale de la phase interne.

La nature et l'épaisseur des excipients en couche externe permettent de contrôler la libération du principe actif trimétazidine en fonction du temps. En particulier, le retardant présent dans la couche externe, i.e. un retardant de la diffusion du principe actif, intervient dans le processus de libération prolongée.

Parmi les retardants utilisables dans les compositions selon l'invention on peut citer à titre indicatif et non limitatif l'éthylcellulose (EC), les dérivés d'éthylcellulose tels que l'acétate de cellulose, l'acétate butyrate de cellulose, l'acétate propionate de cellulose, l'acétate phtalate de cellulose, l'acétate succinate d'hydroxypropylmethylcellulose et/ou les polymétacrylates. Les retardants sont mis en solution organique ou suspension aqueuse au cours de leurs utilisations dans le procédé de fabrication des compositions pharmaceutiques selon l'invention. Parmi les retardants on pourra citer plus particulièrement l'éthylcellulose.

Parmi les anti-agglomérants selon l'invention il peut être cité le talc, les silices et ses dérivés, stéarate de magnésium, acide stéarique et/ou le sodium fumaryl stéarate. L'anti-agglomérant préféré est le talc.

Outre le retardant et l'anti-agglomérant, la couche externe de la composition pharmaceutique selon l'invention comprend un plastifiant. Parmi les plastifiants envisageables selon l'invention, on peut citer l'acétyltributylcitrate, triacétate de glycérol, acétyl triéthylcitrate, acétyl citrate d'éthyl, diéthylsébaçate, dibutylsébaçate, phtalate d'éthyl, phtalate dibutyl, polyéthylène glycol (PEG), glycérol et/ou le propylène glycol. Parmi les plastifiants on citera plus particulièrement l'acétyltributylcitrate.

Plus particulièrement le pourcentage d'éthylcellulose est compris entre 5.5% et 8% de la masse totale de la phase interne. Spécifiquement le pourcentage d'éthylcellulose est de 6.5% de la masse totale de la phase interne.

Le pourcentage d'anti-agglomérant dans la composition pharmaceutique est compris entre 25% et 200%, de préférence entre 100% et 200% de la masse du retardant.

Le pourcentage de talc en tant qu'anti-agglomérant dans la composition pharmaceutique est préférentiellement compris entre 100% et 200% de la masse du retardant.

Le pourcentage de plastifiant dans la composition pharmaceutique est compris entre 5% et 50%, de préférence entre 5% et 30% de la masse du retardant.

Le pourcentage d'acétyltributylcitrate en tant que plastifiant dans la composition pharmaceutique est préférentiellement compris entre 5% et 30% de la masse du retardant.

La phase interne des compositions pharmaceutiques selon la présente invention comprend le principe actif trimétazidine enrobé sur un noyau neutre et un liant.

Parmi les liants selon l'invention on citera l'hydroxypropylmethylcellulose (HPMC), l'hydroxypropylcellulose (HPC), la maltodextrine, le polyvinylpyrrolidone (PVP) et/ou la cellulose microcristalline.

Parmi les liants on pourra citer plus particulièrement l'hydroxypropylmethylcellulose habituellement utilisée dans le domaine de la formulation des médicaments. Plus particulièrement, l'hydroxypropylmethylcellulose choisie est de faible viscosité. De préférence, l'hydroxypropylmethylcellulose utilisée est le Pharmacoat™ 606.

La trimétazidine et le liant sont déposés sur un noyau neutre, le tout constituant la phase interne.

Les noyaux ou pellets conventionnellement utilisés peuvent être solubles ou insolubles dans l'eau. Ces noyaux sont des sphères de sucre ou des sphères de saccharose/amidon ou de cellulose microcristalline. La taille des noyaux varient entre 100-1200µm, de préférence entre 300-1000µm et de manière encore préférée entre 710-850µm ; le cas échéant cette taille peut être modifiée si cela s'avère nécessaire.

La trimétazidine sous sa forme de dichlorhydrate de trimétazidine est préférée dans les compositions selon l'invention. La quantité de dichlorhydrate de trimétazidine dans la composition pharmaceutique est préférentiellement de 80mg.

Le pourcentage de liants dans la composition pharmaceutique est compris entre 1% et 15% de la masse totale de la composition. Le pourcentage d'hydroxypropylmethylcellulose en tant que liant dans la composition pharmaceutique est compris entre 1% et 15% de la masse totale de la composition.

Le pourcentage de noyaux neutres dans la composition pharmaceutique est compris entre 15% et 40% de la masse totale de la composition. Le pourcentage de noyaux de type saccharose/amidon est compris entre 15% et 40% de la masse totale de la composition.

Le pourcentage de trimétazidine dans la composition pharmaceutique est compris entre 35% et 70% de la masse totale de la composition.

De préférence la couche externe des compositions pharmaceutiques selon l'invention comprend de 5.5% à 8% d'éthylcellulose de la masse totale de la phase interne, de 5% à 30% d'acétyltributylcitrate de la masse du retardant et de 100% à 200% de talc de la masse du retardant.

De manière également préférée la phase interne des compositions pharmaceutiques selon l'invention comprend de 15% à 40% de noyau neutre, de 35% à 70% de trimétazidine et de 1% à 15% d'hydroxypropymethylcellulose de la masse totale de la composition.

La quantité des éléments de la composition pharmaceutique est de 80mg de dichlorhydrate de trimétazidine, 36.677mg de minigranule neutre, 6.40mg d'hydroxypropylmethylcellulose, 1.2mg d'acétyltributylcitrate, 8mg d'ethylcellulose et 12mg de talc.

Parmi les compositions pharmaceutiques selon l'invention, il sera cité plus particulièrement celles qui conviennent pour l'administration orale et notamment sous forme de gélule.

Le taux de dissolution in vitro de la composition selon l'invention est de 8 à 28% de trimétazidine libérée après 4 heures et de 37 à 57% de trimétazidine libérée après 8 heures et supérieur à 75% de trimétazidine libérée après 24 heures. Cette cinétique de dissolution est choisie de sorte que la concentration plasmatique thérapeutiquement efficace de trimétazidine obtenue in vivo se prolonge pendant 24 heures après l'administration de la composition pharmaceutique.

Selon le procédé de fabrication des compositions pharmaceutiques selon l'invention, des noyaux neutres sont enrobés par des couches successives de principe actif au moyen de turbine d'enrobage, perforée ou non, ou d'appareils à lit d'air fluidisé. Le principe actif, mis sous forme de solution ou suspension, aqueuse ou organique, est pulvérisé sur les noyaux neutres puis séché.

Les minigranules, préparées par l'un ou l'autre des procédés sont ultérieurement enrobées, soit en turbine d'enrobage, perforée ou non, soit en appareil de type lit d'air fluidisé. Les minigranules sont enrobées au moyen d'une solution ou d'une suspension de retardant intervenant dans la diffusion du principe actif et contrôler ainsi la cinétique de libération.

Les minigranules enrobées sont mises en gélule.

A titre d'exemple on peut citer le procédé de fabrication suivant :

Des noyaux neutres composés de saccharose/amidon sont enrobés par des couches successives de solution de chlorhydrate de trimétazidine associée à de l'hydroxypropylmethylcellulose dans un système à lit d'air fluidisé.

L'enrobage des minigranules ainsi préparées est poursuivi dans un appareil à lit d'air fluidisé et au moyen d'une suspension constituée d'éthylcellulose, d'acétributyl citrate et de talc.

Les minigranules enrobées sont conditionnées en gélule en présence de stéarate de magnésium.

La présente invention concerne également les compositions pharmaceutiques selon l'invention utiles dans le traitement prophylactique de l'angine de poitrine, lors des atteintes choriorétiniennes ainsi que pour le traitement des vertiges d'origine vasculaire.

Les exemples ci-dessous illustrent l'invention sans pour autant la limiter.

Exemple 1 : Composition pharmaceutique pour gélule contenant 80mg de trimétazidine

Les minigranules de trimétazidine sont enrobées d'un film à 6.5% d'éthylcellulose.

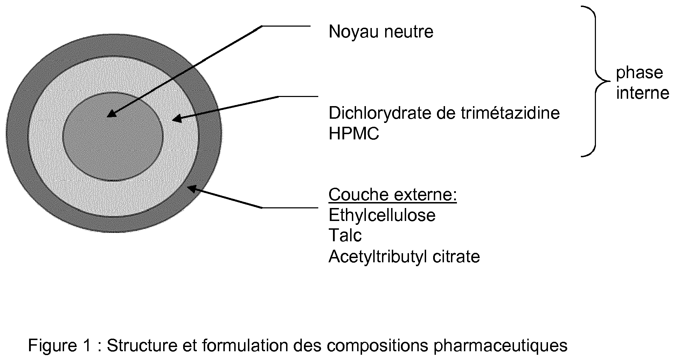

La figure 1 illustre la structure et la formulation de la composition pharmaceutique décrite infra.

Tableau 1 :

| Composés | Quantité (mg) |
|---|---|
| Minigranules de principe actif Dichlorhydrate de trimétazidine Minigranules neutres Hydroxypropylmethylcellulose | 80.00 36.677 6.40 |
| Enrobage (6.5% EC) Acetyltributyl citrate Ethylcellulose Talc | 1.20 8.00 12.00 |
| Gélule Minigranules enrobées Stéarate de magnésium | 144.277 0.434 |

Exemple 2 : Aspect macroscopique des minigranules

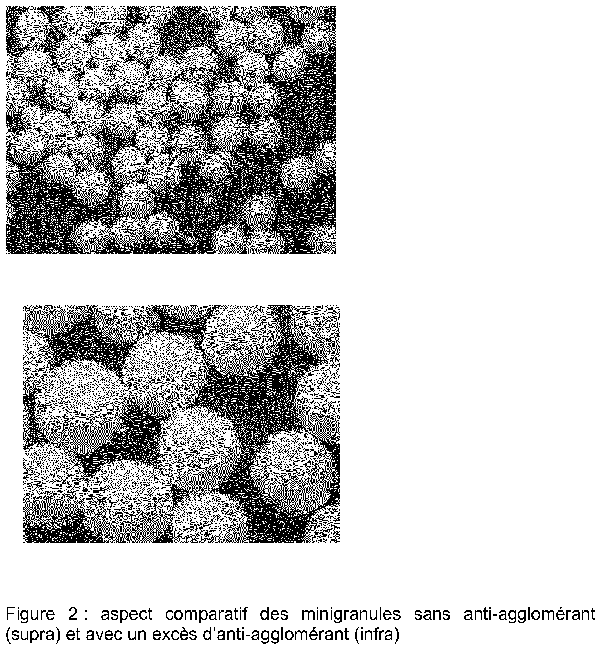

En l'absence d'anti-agglomérant dans la phase externe des compositions pharmaceutiques, on observe à l'échelle industrielle des minigranules irrégulières (Figure 2), de nombreuses fissures dans l'enrobage des minigranules voire des amalgames entre ces dernières. Ces défauts d'enrobage sont à l'origine de modifications majeures des cinétiques de dissolution in vitro en particulier des profils de dissolution accélérée.

Si l'anti-agglomérant est en excès alors les minigranules obtenues à l'échelle industrielle présentent une surface irrégulière et floconneuse (Figure 2) du fait de la présence d'excédent de talc collé à la surface des minigranules. Ces minigranules présentent une cinétique de dissolution très accélérée voire une cinétique à libération immédiate.

Exemple 3 : Cinétiques de dissolution comparées en fonction du pourcentage d'éthylcellulose dans l'enrobage, sachant que seule la composition E110055 correspond aux revendications.

Tableau 2 :

| Lot % EC % ratio enrobage | E110055 6.5 14.7 | E110118 10 20.9 | E110120 4.5 10.7 | E110121 9 19.3 | E110124 5 11.7 |
|---|---|---|---|---|---|
| Composition | | | | | |
| trimétazidine noyaux neutres HPMC | 80 36.677 6.4 | 80 36.677 6.4 | 80 36.677 6.4 | 80 36.677 6.4 | 80 36.677 6.4 |
| éthylcellulose acétyltributyl citrate Talc | 8 1.2 12 | 12.3 1.8 18.5 | 5.5 0.8 8.3 | 11.1 1.7 16.7 | 6.2 0.9 9.3 |
| Stéarate de Mg | 0.4 | 0.4 | 0.4 | 0.4 | 0.4 |
| Masse totale | 144.711 | 156 | 138 | 152.9 | 139.8 |
| Dissolution | | | | | |

(suite)

| Lot | E110055 | E110118 | E110120 | E110121 | E110124 |
|-----|---------|---------|---------|---------|---------|
| 4h | 20.9 | 2.1 | 46.3 | 4.9 | 36.6 |
| 8h | 48.5 | 26.9 | 69.1 | 31.7 | 62.9 |
| 12h | 62.0 | 44.1 | 80 | 48.1 | 75.7 |
| 16h | 72.6 | 54.5 | 86.5 | 58.3 | 83.4 |

Notons que le ratio d'enrobage a été calculé de la manière suivante : masse de la phase externe / masse totale. Par ailleurs, les profils de dissolution ci-dessus ont été obtenus à partir de minigranules non distribuées dans des gélules. Les profils de dissolution des lots à l'échelle industrielle (minigranules encapsulées dans des gélules) sont ralentis de 4, 3, 2 et 1% respectivement aux points 4, 8, 12 et 16 heures par rapport aux profils de dissolution décrits supra (sans conditionnement).

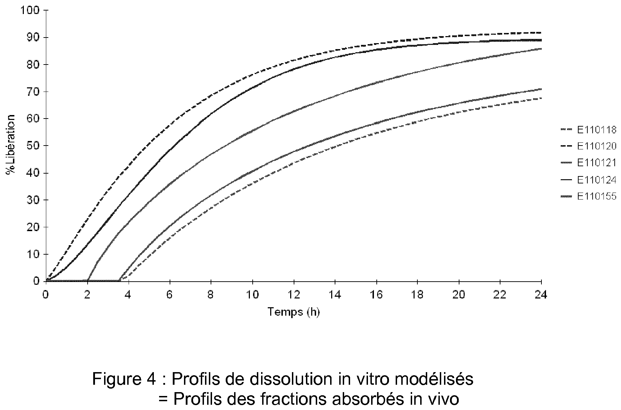

Les cinétiques de dissolution in vitro des compositions pharmaceutiques E110118 (10%EC), E110120 (4.5%EC), E110121 (9%EC) et E110124 (5%EC) (Figure 4) ont été comparées à la cinétique de libération in vitro de la composition pharmaceutique de référence E110055 (6.5%EC). Les profils de dissolution sont comparés à l'aide du facteur de similarité ($f_2$).

Les cinétiques de dissolution des compositions E110118 (10%EC) et E110121 (9%EC) d'une part et E110120 (4.5%EC) et E110124 (5%EC) d'autre part ne sont pas similaires à la cinétique de dissolution de la composition pharmaceutique de référence E110055 (6.5%EC). En conséquence, le pourcentage de retardant dans la composition pharmaceutique est d'une part strictement inférieur à 9% et d'autre part strictement supérieur à 5%.

Deux profils de dissolution sont considérés comme similaires lorsque la valeur ($f_2$) est supérieure ou égale à 50. Le calcul du facteur de similarité ($f_2$) est conseillé par les directives de l'EMA et de la FDA afin de comparer deux profils de dissolution et permettre de conclure à l'identité desdits profils de dissolution.

Le facteur de similarité ($f_2$) a pour formule :

$$f_2 = 50 \bullet \log \left\{ \frac{100}{\sqrt{1 + \dfrac{\sum\limits_{t=1}^{t=n} \left[ \overline{R}(t) - \overline{T}(t) \right]^2}{n}}} \right\}$$

dans laquelle $f_2$ est le facteur de similarité, n est le nombre de points normés, R(t) est la moyenne en pourcentage de principe actif dissout de la composition pharmaceutique de référence E110055 et T(t) est la moyenne en pourcentage de principe actif dissout d'une composition pharmaceutique E110118 (10%EC), E110120 (4.5%EC), E110121 (9%EC) et E110124 (5%EC). Les points normés sont au moins à t=8 heures, t=12 heures et t=16 heures.

Les minigranules remplies en gélules évaluées ont différentes formulations, ces formulations varient en particulier en fonction de la quantité d'éthylcellulose et d'anti-agglomérant.

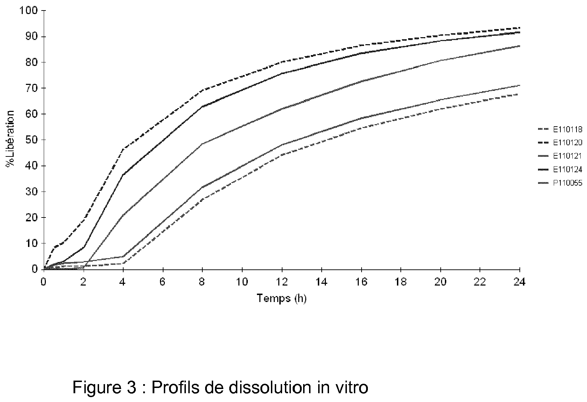

Les profils de dissolution in vitro observés des compositions pharmaceutiques E110118 (10%EC), E110120 (4.5%EC), E110121 (9%EC) et E110124 (5%EC) (Figure 3) ont été modélisés en appliquant pour chacun d'eux la loi de Weibull (Figure 4). La loi de Weibull constitue une approximation particulièrement intéressante permettant de prédire des profils de dissolutions in vitro continus à partir des profils de dissolution in vitro observés.

Une corrélation vitro-vivo égale à 1 a été émise comme hypothèse, dès lors les profils des fractions absorbés in vivo (Figure 4) correspondent exactement aux profils de dissolution in vitro modélisés. Une étape de convolution a ensuite été réalisée pour prédire les profils pharmacocinétiques des différentes compositions pharmaceutiques. L'étape de convolution (fonction Cp(t)) est définie de la manière suivante :

$$Cp(t) = I(t)*P(t)$$

où I(t) est une fonction d'entrée et P(t) est une fonction de disposition.

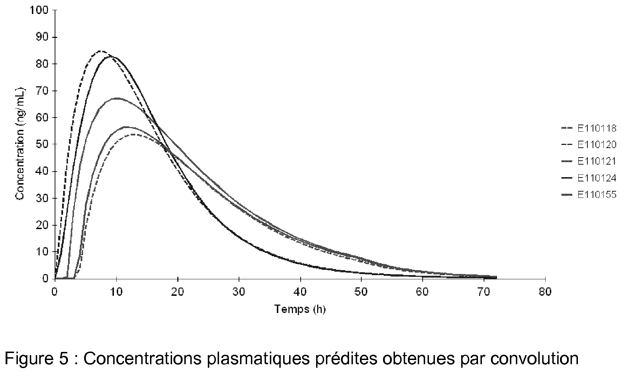

La fonction d'entrée représente les fractions absorbées in vivo en fonction du temps et la fonction de disposition est une équation polyexponentielle de la pharmacocinétique d'une composition pharmaceutique à libération prolongée de trimétazidine obtenue dans l'étude SKH-6790-005-FRA. Les profils pharmacocinétiques moyens prédits sont représentés Figure 5.

A partir desdits profils plasmatiques obtenus par convolution, les paramètres pharmacocinétiques AUC et Cmax ont été calculés où l'AUC représente l'exposition au médicament et Cmax la concentration maximale.

Tableau 3 :

|  | AUC(ng.h/ mL) | Cmax(ng/ mL) |
|---|---|---|
| E110124 | 1620 | 83.6 |
| E110120 | 1678 | 85.6 |
| **E110055** | **1776** | **67.8** |
| E110121 | 1501 | 57.0 |
| E110118 | 1418 | 54.2 |

L'AUC de la composition pharmaceutique E110055 (6.5%EC) est la plus élevée des compositions pharmaceutiques évaluées supra et l'exposition du patient à la trimétazidine est significativement améliorée.

Par ailleurs, les profils de pharmacocinétiques permettent de mesurer le temps de protection myocardique efficace du patient traité soit le temps durant lequel ledit patient est couvert à une concentration plasmatique thérapeutiquement efficace (40µg/l). Ce temps de protection thérapeutique est d'au moins 22 heures pour la composition E110155 (6.5%EC) alors qu'il n'est que de 16 heures pour la composition E110118 (10%EC). Le temps de protection thérapeutique est amélioré alors même que le pourcentage de retardant, responsable dans la composition thérapeutique de la libération prolongée, est diminué de 10% à 6.5%.

**Revendications**

1. Composition pharmaceutique à libération prolongée de trimétazidine dans laquelle :

   - une phase interne comprend un noyau neutre enrobé de trimétazidine ;
   - une couche externe comprend un retardant et un anti-agglomérant

   **caractérisée en ce que** le pourcentage de retardant est compris entre 5,5 et 8% de la masse totale de la phase interne.

2. Composition selon la revendication 1, **caractérisée en ce que** le retardant est sélectionné parmi l'éthylcellulose, acétate de cellulose, acétate butyrate de cellulose, acétate propionate de cellulose, acétate phtalate de cellulose, l'acétate succinate d'hydroxypropylmethylcellulose et/ou les polymétacrylates.

3. Composition selon la revendication 1 ou 2, **caractérisée en ce que** l'anti-agglomérant est sélectionné parmi le talc, dioxide de silice colloïdale, stéarate de magnésium, acide stéarique et/ou sodium fumaryl stéarate.

4. Composition selon l'une quelconque des revendications 1 à 3, **caractérisée en ce que** la couche externe comprend un plastifiant.

5. Composition selon la revendication 4, **caractérisée en ce que** le plastifiant est sélectionné parmi l'acétyltributyl citrate, triacétate de glycérol, acétyl triéthylcitrate, acétyl citrate d'éthyl, diéthylsébaçate, dibutylsébaçate, phtalate d'éthyl et de dibutyl, polyéthylène glycol, glycérol et/ou propylène glycol.

6. Composition selon la revendication 4 ou 5, **caractérisée en ce que** le pourcentage de plastifiant est compris entre 5 et 50% de la masse du retardant.

7. Composition selon l'une quelconque des revendications 1 à 6, **caractérisée en ce que** le pourcentage d'anti-agglomérant est compris entre 25% et 200% de la masse du retardant.

8. Composition selon l'une quelconque des revendications 1 à 7, **caractérisée en ce que** la phase interne comprend un liant.

9. Composition selon la revendication 8, **caractérisée en ce que** le liant est sélectionné parmi l'hydroxypropylmethylcellulose, l'hydroxypropylcellulose, la maltodextrine, le polyvinylpyrrolidone et/ou la cellulose microcristalline.

10. Composition selon l'une quelconque des revendications 1 à 9, **caractérisée en ce que** le noyau neutre est composée de saccharose, saccharose et amidon ou cellulose microcristalline.

11. Composition selon l'une quelconque des revendications 1 à 10, **caractérisée en ce que** la couche externe comprend :

   - de 5% à 30% d'acétyltributylcitrate de la masse du retardant ;
   - de 5.5% à 8% d'éthylcellulose de la masse totale de la phase interne ;
   - de 100% à 200% de talc de la masse du retardant.

12. Composition selon l'une quelconque des revendications 1 à 11, **caractérisée en ce que** la phase interne comprend :

   - de 15% à 40% de noyau neutre de la masse totale de la composition ;
   - de 35% à 70% de trimétazidine de la masse totale de la composition ;
   - de 1% à 15% d'hydroxypropymethylcellulose de la masse totale de la composition.

13. Composition selon l'une quelconque des revendications 1 à 12, **caractérisée en ce que** la trimétazidine se trouve sous la forme de dichlorhydrate.

14. Composition selon l'une quelconque des revendications 1 à 13, **caractérisée en ce qu'**elle contient 80mg de dichlorhydrate de trimétazidine.

15. Composition selon la revendication 1, **caractérisée en ce qu'**elle contient 80mg de dichlorhydrate de trimétazidine, 36.677mg de minigranule neutre, 6.40mg d'hydroxypropylmethylcellulose, 1.2mg d'acétyltributylcitrate, 8mg d'ethylcellulose et 12mg de talc.

16. Composition pharmaceutique selon l'une quelconque des revendications 1 à 15, **caractérisée en ce que** le taux de dissolution in vitro de la composition est de 8 à 28% de trimétazidine libérée après 4 heures et de 37 à 57% de trimétazidine libérée après 8 heures et supérieur à 75% de trimétazidine libérée après 24 heures et choisi de sorte que la concentration plasmatique thérapeutiquement efficace de trimétazidine obtenue in vivo se prolonge pendant 24 heures après l'administration de la composition pharmaceutique.

17. Procédé de fabrication d'une composition selon l'une quelconque des revendications 1 à 16, **caractérisé en ce qu'**il comprend les étapes suivantes :

   a) l'enrobage de noyaux neutres par de la trimétazidine et un liant ;
   b) l'enrobage par un retardant, un plastifiant et un anti-agglomérant des minigranules obtenues en a) ;
   c) le conditionnement des minigranules enrobées obtenues en b) et lubrifiées.

18. Composition pharmaceutique selon l'une quelconque des revendications 1 à 16 utile pour le traitement prophylactique de l'angine de poitrine, lors des atteintes choriorétiniennes ainsi que pour le traitement des vertiges d'origine vasculaire.


**Patentansprüche**

1. Pharmazeutische Zubereitung mit verlängerter Freisetzung von Trimetazidin, worin:

   - eine innere Phase einen von Trimetazidin umhüllten neutralen Kern umfasst;
   - eine äußere Schicht ein Verzögerungsmittel und ein Anti-Agglomerierungsmittel umfasst

   **dadurch gekennzeichnet, dass** der Prozentsatz des Verzögerungsmittels zwischen 5,5 und 8 % der Gesamtmasse der inneren Phase liegt.

**2.** Zubereitung gemäß Anspruch 1, **dadurch gekennzeichnet, dass** das Verzögerungsmittel ausgewählt ist aus Ethylcellulose, Celluloseacetat, Celluloseacetatbutyrat, Celluloseacetatpropionat, Celluloseacetatphthalat, Hydroxypropylmethylcelluloseacetatsuccinat und/oder Polymetacrylaten.

**3.** Zubereitung nach Anspruch 1 oder 2, **dadurch gekennzeichnet, dass** das Anti-Agglomerierungsmittel ausgewählt ist aus Talkum, kolloidalem Siliciumdioxid, Magnesiumstearat, Stearinsäure und/oder Natriumfumarylstearat.

**4.** Zubereitung nach irgendeinem der Ansprüche 1 bis 3, **dadurch gekennzeichnet, dass** die äußere Schicht einen Weichmacher umfasst.

**5.** Zubereitung nach Anspruch 4, **dadurch gekennzeichnet, dass** der Weichmacher ausgewählt ist aus Acetyltributylcitrat, Glycerintriacetat, Acetyltriethylcitrat, Acetylethylcitrat, Diethylsebacat, Dibutylsebacat, Dibutylethylphthalat, Polyethylenglykol, Glycerin und/oder Propylenglykol.

**6.** Zubereitung nach Anspruch 4 oder 5, **dadurch gekennzeichnet, dass** der Prozentsatz des Weichmachers zwischen 5 und 50 % der Masse des Verzögerungsmittels liegt.

**7.** Zubereitung nach irgendeinem der Ansprüche 1 bis 6, **dadurch gekennzeichnet, dass** der Prozentsatz des Anti-Agglomerierungsmittels zwischen 25 % und 200 % der Masse des Verzögerungsmittels liegt.

**8.** Zubereitung nach irgendeinem der Ansprüche 1 bis 7, **dadurch gekennzeichnet, dass** die innere Phase ein Bindemittel umfasst.

**9.** Zubereitung nach Anspruch 8, **dadurch gekennzeichnet, dass** das Bindemittel ausgewählt ist aus Hydroxypropylmethylcellulose, Hydroxypropylcellulose, Maltodextrin, Polyvinylpyrrolidon und/oder mikrokristalliner Cellulose.

**10.** Zubereitung nach irgendeinem der Ansprüche 1 bis 9, **dadurch gekennzeichnet, dass** der neutrale Kern aus Saccharose, Saccharose und Stärke oder mikrokristalliner Cellulose gebildet ist.

**11.** Zubereitung nach irgendeinem der Ansprüche 1 bis 10, **dadurch gekennzeichnet, dass** die äußere Schicht:

- 5 % bis 30 % der Masse des Verzögerungsmittels Acetylbutylcitrat;
- 5,5 % bis 8 % der Gesamtmasse der inneren Phase Ethylcellulose;
- 100 % bis 200 % der Masse des Verzögerungsmittels Talkum umfasst.

**12.** Zubereitung nach irgendeinem der Ansprüche 1 bis 11, **dadurch gekennzeichnet, dass** die innere Phase:

- 15 % bis 40 % der Gesamtmasse der Zubereitung des neutralen Kerns;
- 35 % bis 70 % der Gesamtmasse der Zubereitung Trimetazidin;
- 1 % bis 15 % der Gesamtmasse der Zubereitung Hydroxypropylmethylcellulose umfasst.

**13.** Zubereitung nach irgendeinem der Ansprüche 1 bis 12, **dadurch gekennzeichnet, dass** Trimetazidin in Form des Dihydrochlorids vorliegt.

**14.** Zubereitung nach irgendeinem der Ansprüche 1 bis 13, **dadurch gekennzeichnet, dass** sie 80 mg Trimetazidin-Dihydrochlorid enthält.

**15.** Zubereitung nach Anspruch 1, **dadurch gekennzeichnet, dass** sie 80 mg Trimetazidin-Dihydrochlorid, 36,677 mg neutrales Minigranulat, 6,40 mg Hydroxypropylmethylcellulose, 1,2 mg Acetyltributylcitrat, 8 mg Ethylcellulose und 12 mg Talkum enthält.

**16.** Pharmazeutische Zubereitung nach irgendeinem der Ansprüche 1 bis 15, **dadurch gekennzeichnet, dass** die in vitro-Auflösungsrate der Zubereitung 8 bis 28 % nach 4 Stunden freigesetztes Trimetazidin und 37 bis 57 % nach 8 Stunden freigesetztes Trimetazidin und mehr als 75 % nach 24 Stunden freigesetztes Trimetazidin beträgt und derart ausgewählt ist, dass die in vivo erzielte therapeutisch wirksame Plasmakonzentration von Trimetazidin sich auf 24 Stunden nach der Verabreichung der pharmazeutischen Zubereitung verlängert.

**17.** Verfahren zur Herstellung einer Zubereitung nach irgendeinem der Ansprüche 1 bis 16, **dadurch gekennzeichnet,**

**dass** es die folgenden Stufen umfasst:

a) Umhüllung von neutralen Kernen mit Trimetazidin und einem Bindemittel;
b) Umhüllung der gemäß a) erhaltenen Minigranulate mit einem Verzögerungsmittel, einem Weichmacher und einem Anti-Agglomerierungsmittel;
c) Konditionierung der gemäß b) erhaltenen und mit Gleitmittel versehenen Minigranulate.

18. Pharmazeutische Zubereitung nach irgendeinem der Ansprüche 1 bis 16 zur prophylaktischen Behandlung von Angina pectoris, bei chorioretinalen Vorfällen und bei der Behandlung von Schwindel vaskulären Ursprungs.

**Claims**

1. Pharmaceutical composition for prolonged release of trimetazidine, wherein:

- an inner phase comprises a neutral core coated with trimetazidine;
- an outer layer comprises a retardant and an anti-agglomerant,

**characterised in that** the percentage of retardant is between 5.5 and 8 % inclusive of the total weight of the inner phase.

2. Composition according to claim 1, **characterised in that** the retardant is selected from ethylcellulose, cellulose acetate, cellulose acetate butyrate, cellulose acetate propionate, cellulose acetate phthalate, hydroxypropyl methylcellulose acetate succinate and/or polymethacrylates.

3. Composition according to claim 1 or 2, **characterised in that** the anti-agglomerant is selected from talc, colloidal silicon dioxide, magnesium stearate, stearic acid and/or sodium fumaryl stearate.

4. Composition according to any one of claims 1 to 3, **characterised in that** the outer layer comprises a plasticiser.

5. Composition according to claim 4, **characterised in that** the plasticiser is selected from acetyl tributyl citrate, glycerol triacetate, acetyl triethyl citrate, acetyl ethyl citrate, diethyl sebacate, dibutyl sebacate, ethyl and dibutyl phthalate, polyethylene glycol, glycerol and/or propylene glycol.

6. Composition according to claim 4 or 5, **characterised in that** the percentage of plasticiser is between 5 and 50 % inclusive of the weight of the retardant.

7. Composition according to any one of claims 1 to 6, **characterised in that** the percentage of anti-agglomerant is between 25 % and 200 % inclusive of the weight of the retardant.

8. Composition according to any one of claims 1 to 7, **characterised in that** the inner phase comprises a binder.

9. Composition according to claim 8, **characterised in that** the binder is selected from hydroxypropyl methylcellulose, hydroxypropylcellulose, maltodextrin, polyvinylpyrrolidone and/or microcrystalline cellulose.

10. Composition according to any one of claims 1 to 9, **characterised in that** the neutral core is composed of sucrose, sucrose and starch, or microcrystalline cellulose.

11. Composition according to any one of claims 1 to 10, **characterised in that** the outer layer comprises:

- from 5 % to 30 % of acetyl tributyl citrate relative to the weight of the retardant;
- from 5.5 % to 8 % of ethylcellulose relative to the total weight of the inner phase;
- from 100 % to 200 % of talc relative to the weight of the retardant.

12. Composition according to any one of claims 1 to 11, **characterised in that** the inner phase comprises:

- from 15 % to 40 % of neutral core relative to the total weight of the composition;
- from 35 % to 70 % of trimetazidine relative to the total weight of the composition;

- from 1 % to 15 % of hydroxypropyl methylcellulose relative to the total weight of the composition.

13. Composition according to any one of claims 1 to 12, **characterised in that** the trimetazidine is in the form of the dihydrochloride.

14. Composition according to any one of claims 1 to 13, **characterised in that** it contains 80 mg of trimetazidine dihydrochloride.

15. Composition according to claim 1, **characterised in that** it contains 80 mg of trimetazidine dihydrochloride, 36.677 mg of neutral minigranules, 6.40 mg of hydroxypropyl methylcellulose, 1.2 mg of acetyl tributyl citrate, 8 mg of ethylcellulose and 12 mg of talc.

16. Pharmaceutical composition according to any one of claims 1 to 15, **characterised in that** the level of *in vitro* dissolution of the composition is from 8 % to 28 % of the trimetazidine released by 4 hours and from 37 % to 57 % of the trimetazidine released by 8 hours and more than 75 % of the trimetazidine released by 24 hours and is selected so that the therapeutically efficacious plasma concentration of trimetazidine obtained *in vivo* is prolonged over 24 hours following administration of the pharmaceutical composition.

17. Process for manufacture of a composition according to any one of claims 1 to 16, **characterised in that** it comprises the following steps:

   a) coating neutral cores with trimetazidine and a binder;
   b) coating the minigranules obtained in a) with a retardant, a plasticiser and an anti-agglomerant;
   c) filling of the coated minigranules obtained in b) and lubricated.

18. Pharmaceutical composition according to any one of claims 1 to 16 for use in the prophylactic treatment of angina pectoris, in the course of chorioretinal disorders and also for the treatment of vertigo of vascular origin.

Noyau neutre

phase
interne

Dichlorydrate de trimétazidine
HPMC

Couche externe:
Ethylcellulose
Talc
Acetyltributyl citrate

Figure 1 : Structure et formulation des compositions pharmaceutiques

Figure 2 : aspect comparatif des minigranules sans anti-agglomérant (supra) et avec un excès d'anti-agglomérant (infra)

Figure 3 : Profils de dissolution in vitro

Figure 4 : Profils de dissolution in vitro modélisés
= Profils des fractions absorbés in vivo

Figure 5 : Concentrations plasmatiques prédites obtenues par convolution

## RÉFÉRENCES CITÉES DANS LA DESCRIPTION

**Documents brevets cités dans la description**

- FR 2490963
- EP 1108424 A
- EP 0673649 A